# EUROPÄISCHE PATENTSCHRIFT

(11) **EP 0 576 651 B1**
(45) Veröffentlichungstag und Bekanntmachung des Hinweises auf die Patenterteilung: **06.03.1996**
(21) Anmeldenummer: 93901682.0
(22) Anmeldetag: 13.01.1993
(51) Int. Cl.: A61B 17/60, A61B 17/70

(54) **SPONDYLODESE-IMPLANTAT**
SPONDYLODESIS IMPLANT
IMPLANT POUR SPONDYLODESE

(30) Priorität: 16.01.1992 DE 4200904
(43) Veröffentlichungstag der Anmeldung: 05.01.1994
(73) Patentinhaber: ULRICH, Heinrich, D-89077 Ulm/Donau (DE)
(72) Erfinder: ULRICH, Heinrich, D-89077 Ulm/Donau (DE)
(74) Vertreter: Fay, Hermann, Dipl.-Phys. Dr.
(86) Internationale Anmeldenummer: DE9300031
(87) Internationale Veröffentlichungsnummer: WO9313722

(56) Entgegenhaltungen:
- EP-A- 0 443 894
- DE-A- 3 132 520

## Beschreibung

Die Erfindung betrifft ein Spondylodese-Implantat zur Korrektur und Fixierung der gegenseitigen Wirbelstellung, mit zwei in Längsrichtung der Wirbelsäule an Gewindespindeln geführten und bezüglich ihres Abstandes voneinander verstellbaren Anschlußstücken, und mit in die Wirbel einschraubbaren Repositionsschrauben, für die zur Befestigung der Anschlußstücke an den Wirbeln Aufnahmen in den Anschlußstücken vorgesehen sind.

Aus der DE 31 32 520 A1 bekannte Spondylodese-Implantate dieser Art dienen zur einstellbaren Fixierung zweier unmittelbar benachbarter Wirbel. Die den beiden Anschlußstücken zugeordneten Gewindespindeln sind in koaxialer Anordnung einstückig miteinander ausgebildet, wobei zwischen den beiden Gewindespindeln ein sie verbindendes Außensechskantstück vorgesehen ist, das zum Ansetzen eines Verdrehwerkzeuges zum gemeinsamen Drehen der Gewindespindeln dient. Die Gewindespindeln und entsprechend die Muttergewinde, mit welchen die Anschlußstücke auf ihrer jeweiligen Gewindespindel geführt sind, besitzen entgegengesetzten Gewindesinn, so daß beim gemeinsamen Verdrehen der Gewindespindeln je nach Drehrichtung die beiden Anschlußstücke aufeinander zu- oder voneinander fortbewegt werden.

Der Erfindung liegt die Aufgabe zugrunde, ein Spondylodese-Implantat der eingangs genannten Art so auszubilden, daß drei aufeinander folgende Wirbel relativ zueinander ausgerichtet und fixiert werden können und daß dabei der gegenseitigen Versetzung der Wirbel, soweit sie auf der natürlichen sagittalen Biegung der Wirbelsäule beruhen, in einfacher Weise Rechnung getragen werden kann.

Diese Aufgabe wird bei einem Implantat der eingangs genannten Art erfindungsgemäß dadurch gelöst, daß zwischen den beiden Anschlußstücken ein weiteres Anschlußstück mit ebenfalls einer Aufnahme für eine Repositionsschraube vorgesehen ist und an diesem mittleren Anschlußstück die Gewindespindeln für die beiden äußeren Anschlußstücke unabhängig voneinander drehbar und axial unverschiebbar gelagert sind, wobei die beiden Gewindespindeln in der ihren Achsen gemeinsamen Ebene zwecks Anpassung des Implantats an die sagittale Biegung der Wirbelsäule gegeneinander geneigt sind. Vorzugsweise beträgt die gegenseitige Neigung der Gewindespindelachsen etwa 15°.

Wird ein solches Implantat mit je einem seiner Anschlußstücke an drei aufeinander folgenden Wirbeln befestigt, können durch Verdrehen der Gewindespindeln die beiden äußeren Wirbel unabhängig voneinander gegenüber dem mittleren Wirbel eingestellt und fixiert werden, wobei die gegenseitige Neigung der beiden Gewindespindeln der natürlichen Biegung der Wirbelsäule im Bereich der durch das Implantat miteinander verbundenen drei Wirbel Rechnung trägt.

Eine in konstruktiver Hinsicht bevorzugte Ausführungsform des Implantats ist dadurch gekennzeichnet, daß die Gewindespindeln mit je einem Lagerzapfen in einer Lageraufnahme des mittleren Anschlußstückes geführt sind und am Lagerzapfen eine zur Spindelachse koaxiale Ringnut aufweisen, in die ein anschlußstückseitiger, die Gewindespindel gegen Axialverschiebungen sichernder Vorsprung greift. Dieser Vorsprung ist zweckmäßigerweise von einem im Anschlußstück eingesteckten, tangential in die Ringnut greifenden Querstift gebildet. Es empfiehlt sich, daß jede Gewindespindel zwischen ihrem Gewindeschaft und dem sie lagernden Anschlußstück mit Schlüsselflächen zum Ansetzen eines Verdrehwerkzeugs ausgestattet ist. Zweckmäßigerweise bilden die Schlüsselflächen an jeder Gewindespindel einen mit der Gewindespindel einstückigen Außensechskant.

Die Repositionsschrauben können übliche, mit einem Schraubenkopf versehene Knochenschrauben sein, wobei der Schraubenkopf bei angezogener Schraube am Anschlußstück zur Auflage kommt. Häufiger aber wird man im Rahmen der Erfindung kopflose Repositionsschrauben mit einem an den Gewindeschaft anschließenden glatten Schaftteil verwenden, der nach Eindrehen der Schraube in die jeweiligen Wirbel auf die jeweils für die Befestigung der Anschlußstücke noch benötigte Länge gekürzt werden kann. Besonders für diesen Fall empfiehlt es sich, im Rahmen der Erfindung die Anordnung so zu treffen, daß die Repositionsschrauben in ihren Aufnahmen im wesentlichen parallel zu der den Achsen beider Gewindespindeln gemeinsamen Ebene verlaufen, wobei die Repositionsschrauben in ihrer jeweiligen Aufnahme in Schraubenlängsrichtung verschiebbar und quer dazu verschwenkbar geführt sind, und daß die Aufnahmen mit einer durch ein Klemmglied betätigbaren Klemmeinrichtung ausgestattet sind, die im Klemmzustand die Repositionsschraube sowohl gegen Verschieben als auch Verschwenken in der Aufnahme feststellt.

Im folgenden wird die Erfindung an einem in der Zeichnung dargestellten Ausführungsbeispiel näher erläutert; es zeigen:
- Fig. 1: eine Seitenansicht eines Spondylodese-Implantates nach der Erfindung,
- Fig. 2: eine Draufsicht auf das Implantat nach Fig. 1,

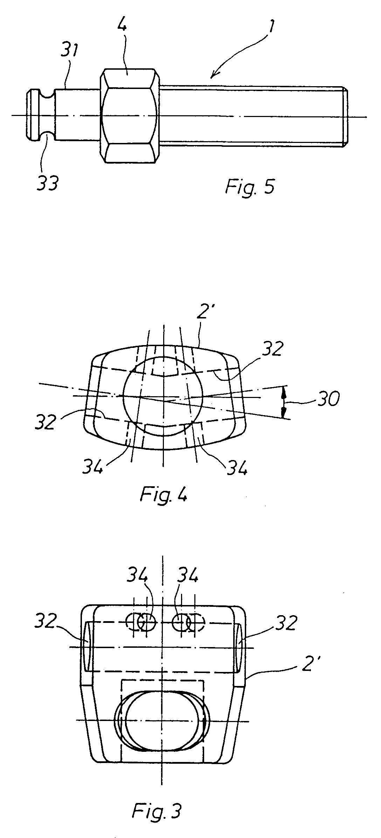

- Fig. 3: eine Draufsicht nur auf das mittlere Anschlußstück des Implantats nach den Fig. 1 und 2 in vergrößerter Darstellung,
- Fig. 4: die Seitenansicht des Anschlußstücks nach Fig. 3,
- Fig. 5: eine der Gewindespindeln des Implantats nach den Fig. 1 und 2 in Seitenansicht,

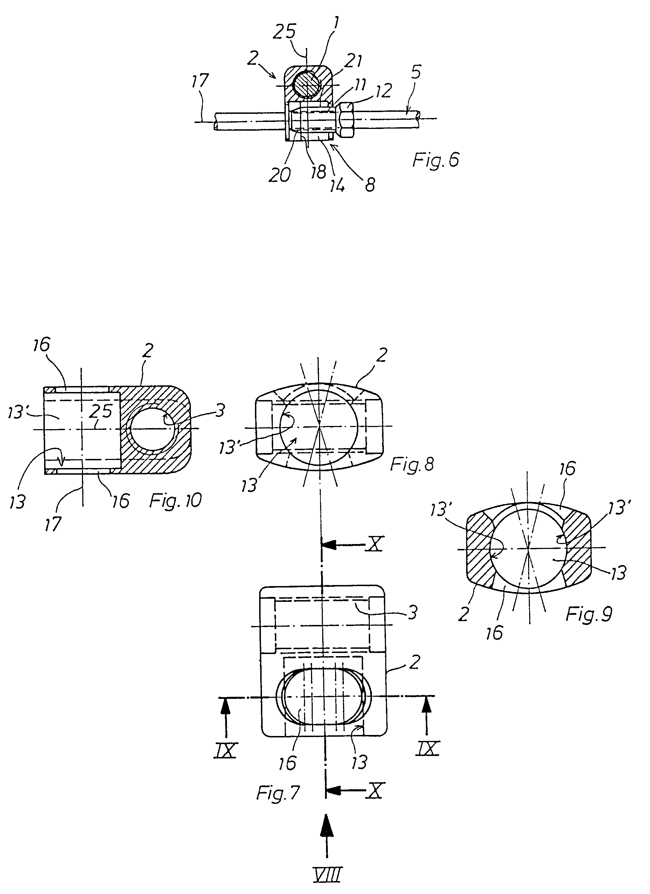

- Fig. 6: einen Schnitt in Richtung IV - IV durch das Implantat nach den Fig. 1 und 2,
- Fig. 7: eine Draufsicht nur auf das Anschlußstück des Gegenstandes der Fig. 6,
- Fig. 8: eine Stirnansicht des Anschlußstücks der Fig. 7 in Richtung des dort eingetragenen Pfeiles VIII,
- Fig. 9: den Schnitt IX - IX in Fig. 7,
- Fig. 10: den Schnitt X - X in Fig. 7,

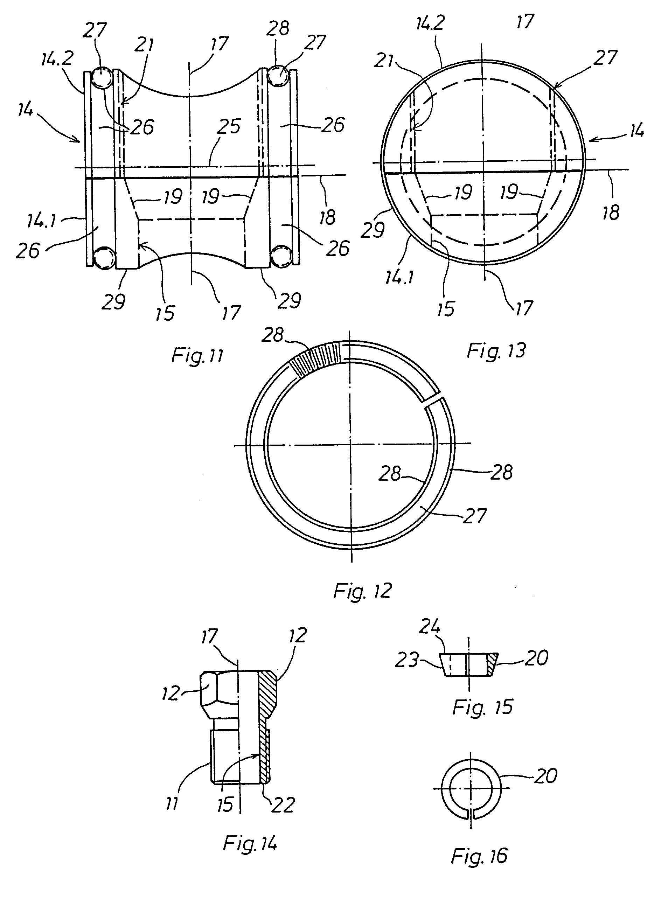

- Fig. 11: den Gelenkkörper des Gegenstandes der Fig. 6 zusammen mit den Federringen in einer Seitenansicht,
- Fig. 12: die Federringe in Fig. 11 in axialer Ansicht,
- Fig. 13: den Gelenkkörper der Fig. 11 in axialer Ansicht,
- Fig. 14: die Gewindehülse,
- Fig. 15: den Klemmring jeweils des Gegenstandes der Fig. 6 teils in Seitenansicht und teils im Schnitt, und
- Fig. 16: eine Axialansicht des Klemmrings nach Fig. 15.

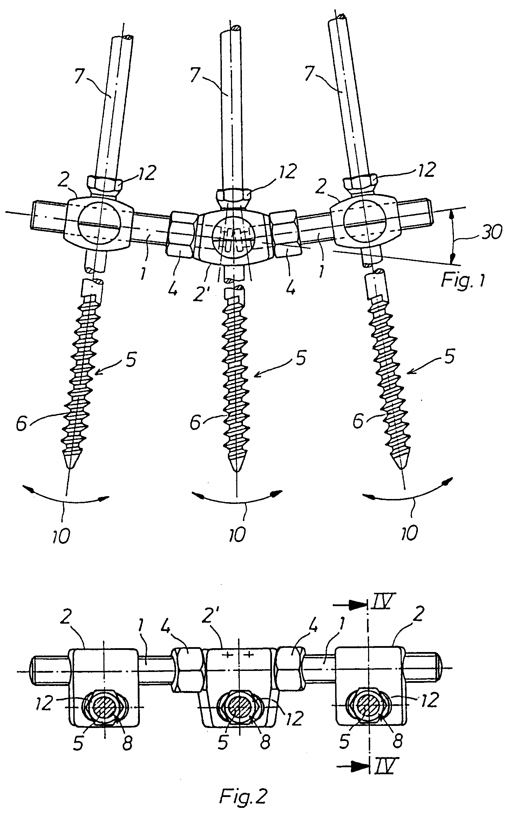

Die Fig. 1 und 2 zeigen ein Spondylodese-Implantat, bei dem an Gewindespindeln 1 Anschlußstücke 2 geführt sind, die durch Verdrehen der Gewindespindeln 1 in ihrem gegenseitigen Abstand voneinander verstellbar sind. Insgesamt sind drei Anschlußstücke 2, 2' vorgesehen, wobei das mittlere Anschlußstück dazu dient, die beiden seitlichen Gewindespindeln 1 unabhängig voneinander drehbar und axial unverschiebbar zu lagern. Dabei sind die beiden Gewindespindeln 1 in der ihren Achsen gemeinsamen Ebene, die in Fig. 1 mit der Zeichenebene übereinstimmt, zwecks Anpassung des Implantats an die sagittale Biegung der Wirbelsäule gegeneinander um einen Winkel 30 von etwa 15° geneigt. Die Gewindespindeln 1 sind mit je einem Lagerzapfen 31 in einer Lageraufnahme 32 des mittleren Anschlußstückes 2' geführt und am Lagerzapfen 31 mit einer zur Spindelachse koaxialen Ringnut 33 versehen, in die tangential ein Querstift 34 greift, der in eine Bohrung im Anschlußstück 2' eingesteckt ist und die Gewindespindel 1 gegen Axialverschiebungen sichert. Jede Gewindespindel 1 ist zwischen ihrem Gewindeschaft und dem sie lagernden mittleren Anschlußstück 2' mit Schlüsselflächen zum Ansetzen eines Verdrehwerkzeugs ausgestattet, wobei diese Schlüsselflächen an jeder Gewindespindel 1 einen mit der Gewindespindel einstückigen Außensechskant 4 bilden. Zur Verbindung der Anschlußstücke 2, 2' mit jeweils einem in der Zeichnung nicht dargestellten Wirbel dienen Knochenschrauben 5, die mit einem Gewindeabschnitt 6 in den Wirbel eingeschraubt werden und mit einem glatten gewindelosen Schaftabschnitt 7 in einer allgemein mit 8 bezeichneten Aufnahme an jedem Anschlußstück gehalten sind. Diese Aufnahmen 8 für die Repositionsschrauben 5 sind in allen drei Anschlußstücken 2, 2' gleich ausgebildet. Dabei sind die Repositionsschrauben 5 in ihrer jeweiligen Aufnahme 8 in Schraubenlängsrichtung mit dem gewindelosen glatten Schaftteil 7 verschiebbar geführt und quer dazu in Richtung der Pfeile 10 auch verschwenkbar. Im übrigen sind die Aufnahmen 8 mit einer durch ein Klemmglied betätigbaren Klemmeinrichtung ausgestattet, die im Klemmzustand die Repositionsschraube 5 sowohl gegen Verschieben als auch Verschwenken in der Aufnahme 8 feststellt. Das Klemmglied ist eine Gewindehülse 11, die zur Betätigung mittels eines Schlüssels wiederum mit einem Sechskant 12 ausgestattet ist. Im folgenden ist die Aufnahme 8 nur für eines der seitlichen Anschlußstücke 2 beschrieben.

Im einzelnen ist im Anschlußstück 2 eine Lageröffnung 13 für einen Gelenkkörper 14 und im Gelenkkörper 14 ein Führungskanal 15 für die Repositionsschraube 5 ausgebildet. Die Repositonsschraube 5 steht beidseits des Gelenkkörpers 14 durch in der Wandung des Anschlußstücks 2 vorgesehene Fenster 16 aus dem Anschlußstück 2 vor. Der Gelenkkörper 14 ist quer zur Achse 17 des Führungskanals 15 in der Ebene 18 zweigeteilt. In einem 14.1 der beiden Teile 14.1, 14.2 ist ein mit dem Führungskanal 15 koaxialer Kegelsitz 19 ausgebildet, in dem sich ein die Repositionsschraube 5 umschließender, umfangsmäßig geschlitzter Klemmring 20 befindet. Das Klemmglied, also die Gewindehülse 11 verspannt im Klemmzustand einerseits die beiden Gelenkkörperteile 14.1, 14.2 in der Lageröffnung 13 gegen deren Lagerfläche 13' und andererseits den Klemmring 20 im Kegelsitz 19 gegen die Repositionsschraube 5. Das wird in einfacher Weise dadurch erreicht, daß der Kegelsitz 19 mit dem erweiterten Ende im Spalt 18 zwischen beiden Gelenkkörperteilen 14.1, 14.2 mündet und in dem dieser Mündung gegenüber liegenden Gelenkkörperteil 14.2 eine mit dem Führungskanal 15 koaxiale Gewindebohrung 21 vorgesehen ist, in der die einen Abschnitt des Führungskanals 15 bildende Gewindehülse 11 geführt ist. Die Gewindehülse 11 stößt mit dem innerhalb des Gelenkkörpers 14 liegenden Hülsenende 22 gegen den im Kegelsitz 19 befindlichen Klemmring 20 und steht am anderen Hülsenende mit dem dort befindlichen Sechskant 12 durch das Fenster 16 nach außen vor, so daß der Sechskant 12 zur Betätigung der Gewindehülse 11 zugänglich ist. Der Klemmring 20 besitzt eine dem Kegelsitz 19 angepaßte äußere Kegelfläche 23. Der der Gewindehülse 11 zugewandte Rand 24 des Klemmrings 20 besitzt einen mindestens so großen Außendurchmesser wie das Ende 22 die Gewindehülse 11. Im Ergebnis stützt sich die Gewindehülse 11 immer auf dem ihr zugewandten Rand 24 des Klemmrings 20 ab. Wird also die Gewindehülse 11 angezogen, wird der Klemmring 20 im Kegelsitz 19 gegen den gewindelosen Schaftteil 7 der Repositionsschraube 5 verspannt; zugleich werden durch die sich an dem Klemmring 20 abstützende Gewindehülse 11 die beiden Gelenkkörperteile 14.1, 14.2 auseinander gedrückt und dadurch in der Lageröffnung 13 verklemmt.

Der Gelenkkörper 14 und die Lageröffnung 13 sind in den Ausführunsbeispielen zylindrisch mit zur Achse 17 des Führungskanales 15 senkrechter Zylinderachse 25 ausgebildet. Die Verschwenkbarkeit des Gelenkkörpers 14 um diese nur eine einzige Achse 25 genügt im Fall der Ausführungsbeispiele, da die Anschlußstücke 2 selbst um die Achse der sie führenden Gewindespindeln 1 verdrehbar sind und sich daher einer Schrägstellung der Repositionsschrauben 5 im Sinne dieser Verdrehmöglichkeit auf der Gewindespindel 1 anpassen können. Bei bezüglich der Führung der Anschlußstücke 2 anders ausgebildeten Implantaten ist es aber selbstverständlich grundsätzlich möglich, Lageröffnungen und entsprechende Gelenkkörper, beispielsweise Kugelgelenkkörper vorzusehen, die auch eine Verdrehung um zwei zueinander senkrechte Achsen ermöglichen. Der Aufbau einer solchen Aufnahme 8 ist dann allerdings aufwendiger. - Im vorliegenden Fall nur zylindrischer Gelenkkörper 14 besteht eine besonders einfache Möglichkeit, eine sehr wirksame Verklemmung des Gelenkkörpers 14 in der Lageröffnung 13 und damit eine sichere Fixierung der Repositionsschrauben 5 gegen Verschwenken zu erreichen. Dazu sind beidseits des Führungskanals 15 an der äußeren Umfangsfläche des Gelenkkörpers 14 koaxial mit der Zylinderachse 25 verlaufende Ringnuten 26 ausgebildet, in die umfangsmäßig geschlitzte Federringe 27 eingelegt sind, die außenseitig mit einer Querrillierung 28 versehen sind und mit den Rillenstegen radial geringfügig über die zylindrische Umfangsfläche 29 des Gelenkkörpers 14 vorstehen. Diese Querrillierung 28 ist dabei in einfacher Weise durch ein auf den Federring 27 aufgebrachtes Gewinde gebildet. Werden daher bei Betätigung der Gewindehülse 11 die beiden Gelenkkörperteile 14.1, 14.2 auseinander gedrückt, erfolgt deren Verspannung gegen die Lagerfläche 13' der Lageröffnung 13 über die Federringe 27, wobei sich deren Rillenstege einerseits in die Lagerfläche 13' der Lageröffnung 13, andererseits in die Wandung der Ringnuten 26 eingraben.

## Patentansprüche

1. Spondylodese-Implantat zur Korrektur und Fixierung der gegenseitigen Wirbelstellung, mit zwei in Längsrichtung der Wirbelsäule an Gewindespindeln (1) geführten und bezüglich ihres Abstandes voneinander verstellbaren Anschlußstücken (2), und mit in die Wirbel einschraubbaren Repositionsschrauben (5), für die zur Befestigung der Anschlußstücke (2) an den Wirbeln Aufnahmen (8) in den Anschlußstücken (2) vorgesehen sind, dadurch gekennzeichnet, daß zwischen den beiden Anschlußstücken (2) ein weiteres Anschlußstück (2') mit ebenfalls einer Aufnahme (8) für eine Repositionsschraube (5) vorgesehen ist und an diesem mittleren Anschlußstück (2') die Gewindespindeln (1) für die beiden äußeren Anschlußstücke (2) unabhängig voneinander drehbar und axial unverschiebbar gelagert sind, wobei die beiden Gewindespindeln (1) in der ihren Achsen gemeinsamen Ebene zwecks Anpassung des Implantats an die sagittale Biegung der Wirbelsäule gegeneinander geneigt sind.

2. Implantat nach Anspruch 1, dadurch gekennzeichnet, daß die gegenseitige Neigung der Gewindespindelachsen etwa 15° beträgt.

3. Implantat nach Anspruch 1 oder 2, dadurch gekennzeichnet, daß die Gewindespindeln (1) mit je einem Lagerzapfen (31) in einer Lageraufnahme (32) des mittleren Anschlußstückes (2') geführt sind und am Lagerzapfen (31) eine zur Spindelachse koaxiale Ringnut (33) aufweisen, in die ein anschlußstückseitiger, die Gewindespindel (1) gegen Axialverschiebungen sichernder Vorsprung greift.

4. Implantat nach Anspruch 3, dadurch gekennzeichnet, daß der Vorsprung von einem im Anschlußstück (2') eingesteckten, tangential in die Ringnut (33) greifenden Querstift (34) gebildet ist.

5. Implantat nach einemd er Ansprüche 1 bis 4, dadurch gekennzeichnet, daß jede Gewindespindel (1) zwischen ihrem Gewindeschaft und dem sie lagernden Anschlußstück (2') mit Schlüsselflächen zum Ansetzen eines Verdrehwerkzeugs ausgestattet ist.

6. Implantat nach Anspruch 5, dadurch gekennzeichnet, daß die Schlüsselflächen an jeder Gewindespindel (1) einen mit der Gewindespindel einstückigen Außensechskant (4) bilden.

7. Implantat nach einem der Ansprüche 1 bis 6, dadurch gekennzeichnet, daß die Repositionsschrauben (5) in ihren Aufnahmen (8) im wesentlichen parallel zu der den Achsen beider Gewindespindeln (1) gemeinsamen Ebene verlaufen, wobei die Repositionsschrauben (5) in ihrer jeweiligen Aufnahme (8) in Schraubenlängsrichtung verschiebbar und quer dazu verschwenkbar geführt sind, und daß die Aufnahmen (8) mit einer durch ein Klemmglied betätigbaren Klemmeinrichtung ausgestattet sind, die im Klemmzustand die Repositionsschraube (5) sowohl gegen Verschieben als auch Verschwenken in der Aufnahme (8) feststellt.

## Claims

1. A spondylodesis implant for correcting and fixing the mutual position of vertebrae, comprising two connecting portions (2) which are guided in the longitudinal direction of the spinal column on screwthreaded spindles (1) and which are adjustable in respect of their spacing from each other, and repositioning screws (5) which can be screwed into the vertebrae and for which receiving means (8) are provided in the connecting portions (2) for fixing the connecting portions (2) to the vertebrae, characterised in that provided between the two connecting portions (2) is a further connecting portion (2') with also a receiving means (8) for a repositioning screw (5) and the screwthreaded spindles (1) for the two outer connecting portions (2) are mounted on said central connecting portion (2') axially immovably and rotatably independently of each other, wherein the two screwthreaded spindles (1) are inclined relative to each other in the plane common to their axes for the purposes of adaptation of the implant to the sagittal flexing of the spinal column.

2. An implant according to claim 1 characterised in that the mutual inclination of the axes of the screwthreaded spindles is about 15°.

3. An implant according to claim 1 or claim 2 characterised in that the screwthreaded spindles (1) are each guided with a respective mounting trunnion (31) in a mounting receiving means (32) in the central connecting portion (2') and on the mounting trunnion (31) have an annular groove (33) which is coaxial with respect to the spindle axis and into which a projection engages, the projection being on the connecting portion and securing the screwthreaded spindle (1) to prevent axial movements.

4. An implant according to claim 3 characterised in that the projection is formed by a transverse pin (34) which is fitted in the connecting portion (2') and which engages tangentially into the annular groove (33).

5. An implant according to one of claims 1 to 4 characterised in that between its screwthreaded shank and the connecting portion (2') supporting it each screwthreaded spindle (1) is provided with wrench surfaces for fitting a turning tool.

6. An implant according to claim 5 characterised in that the wrench surfaces on each screwthreaded spindle (1) form an outside hexagon (4) which is integral with the screwthreaded spindle.

7. An implant according to one of claims 1 to 6 characterised in that the repositioning screws (5) extend in their receiving means (8) substantially parallel to the plane which is common to the axes of both screwthreaded spindles (1), wherein the repositioning screws (5) are guided in their respective receiving bans (8) slidably in the longitudinal direction of the screw and pivotably transversely relative thereto, and that the receiving means (8) are provided with a clamping device which can be actuated by a clamping member and which in the clamping condition fixes the repositioning screw (5) to prevent both sliding movement and also pivotal movement in the receiving means (8).

## Revendications

1. Implant pour spondylodèse pour corriger et fixer la position réciproque de vertèbres, comportant deux éléments de raccordement (2) qui sont guidés sur des broches filetées (1) dans la direction longitudinale de la colonne vertébrale et dont la distance réciproque est réglable, et comportant des vis de repositionnement (5) pouvant être vissées dans les vertèbres et pour lesquelles des logements (8) sont prévus dans les éléments de raccordement (2) pour la fixation de ces derniers aux vertèbres, caractérisé en ce qu'entre les deux éléments de raccordement (2) est prévu un autre élément de raccordement (2') comportant également un logement (8) pour une vis de repositionnement (5), et que les broches filetées (1) pour les deux autres éléments de raccordement (2) sont montées de manière à pouvoir tourner indépendamment l'une de l'autre, sans possibilité de déplacement axial, sur cet élément de raccordement médian (2'), les deux broches filetées (1) étant inclinées l'une par rapport à l'autre dans le plan commun de leurs axes, pour permettre l'adaptation de l'implant à la flexion sagittale de la colonne vertébrale.

2. Implant selon la revendication 1, caractérisé en ce que l'inclinaison réciproque des axes des broches filetées est égale à environ 15°.

3. Implant selon la revendication 1 ou 2, caractérisé en ce que les broches filetées (1) traversent par un tourillon respectif (31) un logement de support (32) de l'élément de raccordement médian (2') et comportent une gorge annulaire (33) coaxiale à l'axe de la broche et qui est aménagée dans le tourillon (31) et dans laquelle s'engage un appendice saillant situé sur l'élément de raccordement et qui bloque la broche filetée (1) contre des déplacements axiaux.

4. Implant selon la revendication 3, caractérisé en ce que l'appendice saillant est formé par une tige transversale (34) qui est enfichée dans l'élément de raccordement (2') et s'engage tangentiellement dans la gorge annulaire (33).

5. Implant selon l'une des revendications 1 à 4, caractérisé en ce que chaque broche filetée (1) comporte, entre sa tige filetée et l'élément de raccordement (2') qui la supporte, des surfaces d'application d'une clé, permettant l'application d'un outil d'entraînement en rotation.

6. Implant selon la revendication 5, caractérisé en ce que les surfaces d'application d'une clé forment, sur chaque broche filetée (1), un profil hexagonal extérieur (4) d'un seul tenant avec la broche filetée.

7. Implant selon l'une des revendications 1 à 6, caractérisé en ce que les vis de repositionnement (5) s'étendent, dans leurs logements (8), essentiellement parallèlement au plan commun contenant les axes des deux broches filetées (1), les vis de repositionnement (5) pouvant être déplacées dans leurs logements respectifs (8) dans la direction longitudinale des vis et étant guidées de manière à pouvoir pivoter transversalement par rapport à cette direction, et que les logements (8) sont pourvus d'un dispositif de serrage qui peut être actionné par un organe de serrage et qui, à l'état serré, bloque la vis de repositionnement (5) aussi bien contre une translation que, également, contre un basculement dans le logement (8).
